# EUROPEAN PATENT APPLICATION

(11) **EP 0 943 324 A2**
(43) Date of publication of application: **22.09.1999**
(21) Application number: 99400621.1
(22) Date of filing: 12.03.1999
(51) Int. Cl.: A61K 7/48

(54) **External skin treatment composition**

(30) Priority: 17.03.1998 JP 8792998; 28.07.1998 JP 22761498
(71) Applicant: SHISEIDO COMPANY, LTD., Chuo-ku Tokyo (JP)
(72) Inventor: Ito, Akira, c/o Shiseido Beauty Science Center, Tokyo (JP); Yoshida, Yuzo, c/o Shiseido Research Center, Yokohama-shi, Kanagawa 223-8553 (JP); Denda, Mitsuhiro, c/o Shiseido Research Center, Yokohama-shi, Kanagawa (JP); Kawai, Eriko, c/o Shiseido Research Center, Yokohama-shi, Kanagawa 223-8553 (JP)
(74) Representative: Rinuy, Santarelli

(57) **Abstract**

An external skin treatment composition suitable for use in the improvement and prevention of skin roughening or in the promotion of recovery of skin barrier function, containing, as an effective component, a sugar alcohol having 5 or 6 carbon atoms (e.g., xylitol, sorbitol, mannitol) preferably, in an amount of 0.005 to 30% by weight, based upon the total amount of the composition, and a carrier therefor.

## Description

### BACKGROUND OF THE INVENTION

### 1. Field of the Invention

The present invention relates to an external skin treatment composition. More specifically, the present invention relates to an external skin treatment composition suitable for use in the improvement and prevention of skin roughening, which has the effect of improvement and prevention of the rough skin symptom due to contact dermititis, psoriasis, and other various types of skin diseases and also rough skin and chaffing of healthy persons and is superior in feeling of use by formulating a C₅ or C₆ sugar alcohol therein. The present invention also relates to an external skin treatment composition useful for promoting the recovery of the skin barrier function and preventing dyskeratosis etc. due to the decline in the epidermal functions of the skin.

### 2. Description of the Related Art

In the rough skin symptom seen in various skin diseases etc., it is known that there is much more disappearance of moisture from the skin compared with healthy skin. It has been thought that the reduction in components believed to function to retain moisture in the epidermis is related to the increase in the so-called transepidermal water loss (TEWL).

Therefore, in the past, as an effective ingredient exhibiting an effect of improvement or prevention of skin diseases or rough skin, polyol compounds such as glycerol, amino acids as NMF (i.e., natural moisturing factors), and also mucosaccharides such as hyaluronic acid or the similar substances thereof are high in safety, and therefore, has been formulated in cosmetics and skin external treatment compositions from the viewpoint of reinforcing the moisture retention in the skin.

Most conventional effective components may have been superior in their moisture retention, but were not necessarily satisfactory in the effect of improvement and prevention of rough skin. Further, even a substance superior in moisture retention and effect of improvement of rough skin such as glycerol has had the defect that it is often accompanied with a distinctive sticky feeling in use in a preparation containing the same and largely detracts from the value of the product due to its poor usability.

In view of the above situation, the present inventors engaged in a broad search for substances superior in moisture retention in the skin, effective against rough skin, and with a good feeling of use when made into a preparation and as a result formed that erythritol, a C₄ sugar alcohol, is effective for improving rough skin and filed an application for the same (Japanese Unexamined Patent Publication (Kokai) No. 4-124118). However, this was still not satisfactory.

On the other hand, recently, there have been reports that specific substances which stimulate biosynthesis of epidermal components responsible for the skin barrier function have an effect of improving rough skin (Japanese Unexamined Patent Publication (Kokai) No. 9-2952).

Furthermore, it has been reported that when the skin barrier function is decreased, the epidermal function of the skin is decreased and dyskeratosis etc. occur. In particular, in the case of senior citizens, it takes a long time for recovery of a decreased skin barrier function. Therefore, demand of a novel skin barrier function recovery promoter which is effective for prevention of dyskeratosis etc. due to the decrease in the epidermal functions of the skin from the aging has been desired.

But there has not been sufficient research into substances having the effect of improvement or recovery of the skin barrier function and also the relationship between the effect of improvement of the skin barrier function and effect of improvement and prevention of rough skin has not been clarified. Further, substances having an effect of improvement and prevention of rough skin do not necessarily have an effect of improvement of the skin barrier function.

### SUMMARY OF THE INVENTION

Accordingly, the objects of the present invention are to eliminate the above-mentioned problems of the prior art and to provide an external skin treatment composition for improving and preventing roughening of skin having a further superior moisture retention and effect of improvement and prevention of rough skin and superior in feeling of use.

Another object of the present invention is to provide an external skin treatment composition for promoting recovery of the decreased skin barrier function in an extremely short time.

In accordance with the present invention, there is provided an external skin treatment composition comprising, as an effective component, a sugar alcohol having 5 or 6 carbon atoms and a carrier therefor.

### BRIEF DESCRIPTION OF THE DRAWINGS

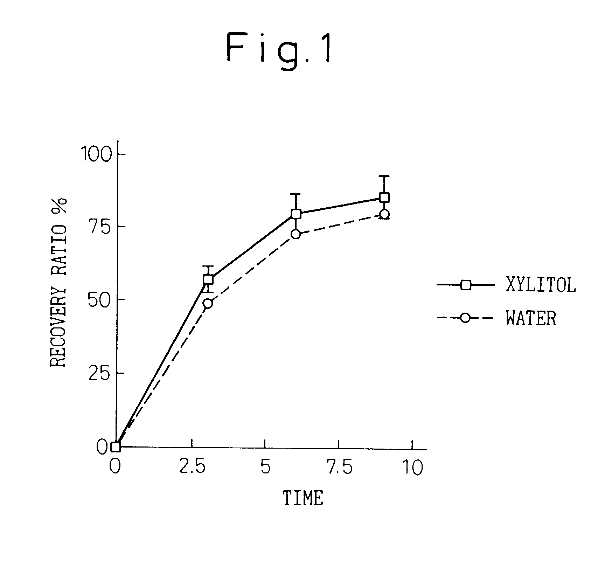

The present invention will be better understood from the description set forth below with reference to the accompanying drawing of Fig. 1, which is a graph evaluating the effect of recovery of the barrier function of skin by xylitol using the TEWL as an indicator.

### DESCRIPTION OF THE PREFERRED EMBODIMENTS

Therefore, the present inventors engaged in further studies and, as a result, found that C₅ or C₆ sugar alcohols such as xylitol, sorbitol, mannitol, especially xylitol, are superior in moisture retention and further have the effect of improvement and prevention of rough skin and that the feeling of use when made into a preparation is good, and further, xylitol promotes recovery of the decreased skin barrier function and thus completed the present invention.

That is, the present invention first provides an external skin treatment composition for improving and preventing roughening skin having a C₅ or C₆ sugar alcohol, as an effective component.

So far as the present inventors know, there have not been any reports up to now of a C₅ or C₆ sugar alcohol being provided with both a superior effect of improvement and prevention of rough skin and a promoting effect of barrier function recovery and further a superior feeling of use.

Applications of sugar alcohols to external skin treatment compositions or cosmetics are described in Japanese Unexamined Patent Publication (Kokai) No. 62-7, Japanese Unexamined Patent Publication (Kokai) No. 3-109309, Japanese Unexamined Patent Publication (Kokai) No. 5-301812, Japanese Unexamined Patent Publication (Kokai) No. 7-252127, etc., but sugar alcohols have mainly only been utilized since they are superior in suppressing the irritability of other effective ingredients, stabilization of the preparations, and feeling of use.

Further, Japanese Unexamined Patent Publication (Kokai) No. 8-508745 describes a preparation using both xylitol and at least one substance selected from mannitol, sorbitol, glycerol, urea, etc., but these were mixed in for the purpose of obtaining a moisturizing and cooling effect when used on the skin or mucous membrane.

The first aspect of the present invention will now be explained below.

The first aspect of the present invention is completed, as a result of the new founding that a C₅ or C₆ sugar alcohol has a particularly superior effect of improvement and prevention of rough skin and is superior in feeling of use by use as the effective compound of an external skin treatment composition for improving rough skin even among sugar alcohols and provides an external skin treatment composition for improving rough skin having a C₅ or C₆ sugar alcohol as an effective component (hereinafter referred to as the external skin treatment composition for improving and preventing roughening of skin according to the present invention).

The effective component of the external skin treatment composition for improving rough skin of the present invention, that is, the C₅ or C₆ sugar alcohol, is a substance having the following chemical formula (I) and is known as an important metabolin serving as a source of energy in the body and also involved in the biosynthesis of nucleic acids and other body components and in the detoxification mechanism in the body.

CH₂OH(CH₂OH)ₙ-CH₂OH (I)

wherein, n is a number of 3 or 4

The C₅ or C₆ sugar alcohol used may be either a natural product or a substance obtained by reducing the corresponding sugar. As typical examples, xylitol may be mentioned as a C₅ substance, while sorbitol and mannitol may be mentioned as C₆ substances.

These sugar alcohols may be used alone or in any combination thereof. The amount formulated in the external skin treatment composition for improving of rough skin of the present invention is preferably 0.005 to 30% by weight, more preferably 0.01 to 10% by weight, based upon the total amount of the external skin treatment composition. If the amount formulated is less than 0.005% by weight, based upon the total amount of the external skin treatment composition, a sufficient effect of improving and preventing rough skin is not exhibited, while even if added over 30% by weight, not that great an improvement of the effect can be seen.

By using a C₅ or C₆ sugar alcohol as an effective component in this way, an external skin treatment composition for improving rough skin of the present invention having the desired particularly superior effect of improving and preventing rough skin and further superior in feeling of use is provided.

The external skin treatment composition for improving rough skin of the present invention may have further added to it, to an extent not adversely affecting the desired effect of the present invention, in addition to the above essential component, the components normally used in cosmetics, pharmaceuticals, or other external skin treatment composition, for example, UV absorbents, moisture retainers other than the above effective ingredient, vitamins, hormones, whitening agents, astringents, refrigerants, various types of extracts, and other medicinal ingredients.

Further, in the external skin treatment composition for improving rough skin of the present invention, it is possible to add a broad range of generally known basic ingredients etc. in accordance with the specific form or preparation to an extent where the addition does not impair the desired effect of the present invention.

That is, it is possible to add to the external skin treatment composition for preventing rough skin of the present invention, a surfactant, liquid oil or fat, liquid or solid oil or fat, solid oil or fat, wax, ester oil, hydrocarbon oil, silicone, sterol, water soluble polymer, chelating agent, neutralizer, pH adjuster, antioxidant, antibacterial, etc.

Further, it is possible to add to the external skin treatment composition for improving rough skin of the present invention, a suitable perfume, dye, etc. if necessary, to an extent not impairing the desired effect of the present invention.

Note that the basic ingredients etc. shown above are examples and that the basic ingredients etc. which may be added to the external skin treatment composition for improving rough skin of the present invention are not limited to these substrate ingredients etc.

These basic ingredients etc. may be suitably combined and added to the external skin treatment composition for improving rough skin of the present invention according to formulations based on the desired form.

The form of the preparation of the external skin treatment composition for preventing rough skin of the present invention is not particularly limited. For example, it may be any of an ointment, cream, emulsion, lotion, pack, bath agent, or other form used for conventional external skin treatment composition.

The present invention further provides an external skin treatment composition for promoting the recovery of barrier function comprising xylitol.

Thus, the second aspect of the present invention will now be explained.

The sugar alcohol usable in the present invention are xylitol, mannitol, sorbitol. The xylitol used in the present invention is a sugar alcohol having the chemical formula (II):

CH₂OH(CHOH)₃CH₂OH (II)

and corresponds to D-xylose. Xylitol is a substance known in the past as a component for external skin treatment compositions or cosmetics, but there have not been any reports up to now of it having an effect of promoting the recovery of the skin barrier function. This effect was first found by the present inventors.

In the second aspect of the present invention,
"promotion of the recovery of the skin barrier function" means that the value of the transepidermal water loss (TEWL) at different measurement times, when the value of the TEWL after one hour after tape stripping of the skin is 0% and the value before the tape stripping is 100%, clearly shows a significant difference when compared with the state where nothing is applied and differs from the so-called effect of improvement of rough skin judged by treating the skin by cotton balls impregnated with 4% sodium dodecyl sulfate (SDS) aqueous solution in accordance with the method of Andrew et al. (J. *Invest. Dermatol,* 86; 598, 1986).

The promoter for recovery of skin barrier function according to the present invention may be formulated into an ointment, cream, emulsion, lotion, pack, bath agent, or other cosmetic, pharmaceutical, or quasi-pharmaceutical and applied to the skin. The amount formulated is not particularly limited, but is preferably 0.005 to 30% by weight, more preferably 0.1 to 20% by weight, based upon the total weight of the composition.

### EXAMPLES

The present invention will now be further illustrated by but is by no means limited to, the following Examples.

Note that the units of amounts formulated in the following Examples are all percents by weight based upon the total amount.

### Examples I-1 - I-11 and Comparative

### Examples I-1 - I-4

First, the method of testing the effect of prevention and improvement of rough skin of the present invention and the results thereof and the method of testing the feeling of use of the preparations and the results thereof will be explained.

### 1. Test of Effect of Prevention of Rough Skin

The effect of prevention of rough skin due to application to the external skin of the present external skin treatment composition for improving rough skin was evaluated using the rough skin caused by a surfactant.

That is, following the method of Andrew et al. (J. *Invest. Dermatol*, 86;598, 1986), using cotton balls impregnated with a 4% sodium dodecyl sulfate (SDS) aqueous solution, five locations at the lower legs (one side) of 30 male panelists were treated by wiping for 30 seconds, then were lightly washed by water and the moisture wiped off. Four types of lotions of the compositions shown in Table I-1 (wt%) were then applied. The above operation was repeated 3 times a day continuously for 2 weeks. At the second week, the states of the skin at the locations coated with the samples were judged visually and ranked based on the judgement criteria shown below. As samples, use was made of one containing xylitol as a product of the present invention and ones containing erythritol, glycerol, or propylene glycol and a control product not containing any of the above ingredients as comparative products. The results are shown in Table I-2.
o Criteria for judgement of effect of prevention of rough skin
   Rank
   0: No dryness or scaling observed
   1: Slight dryness or scaling compared with control product observed
   2: Slight dryness or scaling compared with control product observed
   3: Same degree of dryness or scaling compared with control product observed
o Evaluation of effect of prevention of rough skin
   Very good: At least 80% ratio (effective rate) of test subjects exhibiting rank 0, 1, and 2
   Good: 50 to less than 80% ratio (effective rate) of test subjects exhibiting rank 0, 1, and 2
   Fair: 30 to less than 50% ratio (effective rate) of test subjects exhibiting rank 0, 1, and 2
   Poor: Less than 30% ratio (effective rate) of test subjects exhibiting rank 0, 1, and 2

**Table I-1**

| (wt%) | | | | | |
|---|---|---|---|---|---|
| Sample | Ex. | Comp. Ex. | | | |
| | I-1 | I-1 | I-2 | I-3 | I-4 |
| Xylitol | 10.0 - | | - | - | - |
| Erythritol | - | 10.0 | - | - | - |
| Glycerol | - | - | 10.0 - | | - |
| Propylene glycol | - | - | - | 10.0 - | |
| Ethanol | 7.0 | 7.0 | 7.0 | 7.0 | 7.0 |
| Polyoxyethylene (20 mol) oleyl alcohol | 0.5 | 0.5 | 0.5 | 0.5 | 0.5 |
| Ion exchanged water | Bal. | Bal. | Bal. | Bal. | Bal. |

**Table I-2**

| Sample | Ex. | Comp. Ex. | | | |
|---|---|---|---|---|---|
| | I-1 | I-1 | I-2 | I-3 | I-4 |
| Effect of prevention of rough skin | Very good | Fair | Good | Poor | Poor |

As clear from Table 2, Example 1 of the product of the present invention containing xylitol exhibited a superior effect of prevention from the control product (Comparative Example 4) and Comparative Examples 1 to 3 containing other sugar alcohols or polyol compounds (propylene glycol) with respect to rough skin caused by a surfactant.

### 2. Test of effect of improvement of rough skin and test of usability

### (1) Test of actual use

The effect of improvement of rough skin caused by application to the external skin of the present external skin treatment composition for improving rough skin was evaluated using the rough skin of women in the winter.

That is, the lotions of the compositions (wt%) shown in Table I-1 were applied twice a day continuously for 2 weeks to 100 female panelists suffering from rough skin in the winter - with the product of the present invention applied to one of the left and right cheeks and one of the four types of comparative products applied to the other cheek. The subsequent states of the skin were judged visually. Further, after 2 weeks of use, a questionnaire was given on the feeling of use to judge the quality of the feeling of use. The judgement criteria were as shown below:

The results are shown in Table I-3.
o Criteria of judgement of effect of improvement of rough skin
   Remarkably effective: Disappearance of symptoms observed
   Effective: Weakening of symptoms observed
   Somewhat effective: Some weakening of symptoms observed
   Ineffective: No change in symptoms observed
o Evaluation of effect of improvement of rough skin
   Very good: 80% or more ratio (effective ratio) of test subjects exhibiting "remarkably effective", "effective", and "somewhat effective"
   Good: 50 to less than 80% ratio (effective ratio) of test subjects exhibiting "remarkably effective", "effective", and "somewhat effective"
   Fair: 30 to less than 50% ratio (effective ratio) of test subjects exhibiting "remarkably effective", "effective", and "somewhat effective"
   Poor: Less than 30 ratio (effective ratio) of test subjects exhibiting "remarkably effective", "effective", and "somewhat effective"
o Criteria of judgement of feeling of use
   Very good: Extremely good feeling of use
   Good: Somewhat good feeling of use
   Fair: Somewhat poor feeling of use
   Poor: Extremely poor feeling of use

**Table I-3**

| Sample | Ex. Comp. Ex. | | | | |
|---|---|---|---|---|---|
| | I-1 | I-1 | I-2 | I-3 | I-4 |
| Effect of improvement of rough skin | Very good | Fair | Good | Poor | Poor |
| Feeling of use | Very good | Good | Poor | Good | Poor |

As clear from Table I-3, Example I-1 of the product of the present invention containing xylitol exhibited a superior effect of improvement of rough skin and further a superior feeling of use compared with the control product (Comparative Example 1-4) and Comparative Examples I-1 to I-3 containing other sugar alcohols or polyol compounds.

### (2) Evaluation by replica method

The surface morphology of the skin of healthy women (faces) was observed under a microscope (17X) by taking replicas of the skin using the replica method using Myristin resin. Using 100 subjects judged to have rough skin of ranks 1 or 2 based on the criteria shown in Table 4 from the state of dermatoglyph and desquamation of the corneal layer (rough skin panel), the lotions of the product of the present invention and one of the comparative products I-1 to I-4 of Table I-1 were applied to the face once a day for 2 weeks. After 2 weeks, the states of the skin were observed by the above replica method again and evaluated in accordance with the judgement criteria of Table I-4. The results are shown in Table I-5.

**Table I-4**

| Score | Criteria of evaluation |
|---|---|
| 1 | Sulcus cutis and crista cutis disappear and broad range of desquamation of corneal layer observed. |
| 2 | Sulcus cutis and crista cutis unclear and desquamation of corneal layer observed. |
| 3 | Sulcus cutis and crista cutis observed, but flat. |
| 4 | Sulcus cutis and crista cutis clear. |
| 5 | Sulcus cutis and crista cutis clear and present all over. |

**Table I-5**

| Replica evaluation | Ex. | Comp. Ex. | | | |
|---|---|---|---|---|---|
| | I-1 | I-1 | I-2 | I-3 | I-4 |
| 1 | 0 | 2 | 0 | 4 | 7 |
| 2 | 1 | 3 | 3 | 4 | 5 |
| 3 | 2 | 10 | 8 | 8 | 8 |
| 4 | 9 | 5 | 6 | 4 | 0 |
| 5 | 8 | 0 | 3 | 0 | 0 |

As clear from Table I-5, the lotion of the product of the present invention was observed to have a remarkable effect of improvement of rough skin compared with the lotions of Comparative Examples I-1 to I-4.

Other examples of the external skin treatment composition for improvement of rough skin of the present invention will be given below. All of these were superior in effect of improvement of rough skin and feeling of use.

| Example I-2: Cream | |
|---|---|
| Recipe | Blend ratio |
| Stearic acid | 5.0 |
| Stearyl alcohol | 4.0 |
| Isopropyl myristate | 18.0 |
| Glyceryl monostearic ester | 3.0 |
| Propylene glycol | 10.0 |
| Sorbitol | 0.1 |
| Potassium hydroxide | 0.2 |
| Sodium bisulfite | 0.01 |
| Preservative | q.s. |
| Perfume | q.s. |
| Ion exchanged water | Bal. |

### Preparation Method

The propylene glycol, sorbitol, and potassium hydroxide were added to the ion exchanged water to dissolve and were heated to and held at 70°C (aqueous phase). The rest of the components were mixed, heated to melt, and held at 70°C (oil phase). The oil phase was gradually added to the aqueous phase. The mixture was held at that temperature for a little while after the entire amount was finished being added to cause a reaction. Next, this was homogeneously emulsified by a homomixer and cooled to 30°C while stirring well to obtain a cream.

| Example I-3: Cream | |
|---|---|
| Recipe | Blend ratio |
| Stearic acid | 2.0 |
| Stearyl alcohol | 7.0 |
| Hydrogenated lanolin | 2.0 |
| Squalane | 5.0 |
| 2-Octyldodecyl alcohol | 6.0 |
| Polyoxyethylene (25 mol) cetyl alcohol ether | 3.0 |
| Glycerol monostearic ester | 2.0 |
| Propylene glycol | 5.0 |
| Mannitol | 1.0 |
| Sodium bisulfite | 0.03 |
| Ethyl paraben | 0.3 |
| Perfume | q.s. |
| Ion exchanged water | Bal. |

### Preparation Method

The mannitol and propylene glycol were added to the ion exchanged water which was then heated and held at 70°C (aqueous phase). The rest of the components were mixed and heated to melt and held at 70°C (oil phase). The oil phase was added to the aqueous phase and preemulsified, homogeneously emulsified by a homomixer, then cooled to 30°C while stirring well to obtain a cream.

| Example I-4: Cream | |
|---|---|
| Recipe | Blend ratio |
| Solid paraffin | 5.0 |
| Beeswax | 10.0 |
| Vaseline | 15.0 |
| Liquid paraffin | 41.0 |
| Glyceryl monostearic ester | 2.0 |
| Polyoxyethylene (20 mol) sorbitan monolauric ester | 2.0 |
| Soap powder | 0.1 |
| Sodium borate | 0.2 |
| Xylitol | 3.0 |
| Sodium bisulfite | 0.03 |
| Ethyl paraben | 0.3 |
| Perfume | q.s. |
| Ion exchanged water | Bal. |

### Preparation Method

The xylitol, soap powder, and sodium borate were added to the ion exchanged water, then heated and held at 70°C (aqueous phase). The rest of the components were mixed, heated to melt, and held at 70°C (oil phase). The oil phase was gradually added to the aqueous phase while stirring for the reaction. Next, the resultant mixture was homogeneously emulsified by a homomixer, then cooled to 30°C, while stirring well to obtain a cream.

| Example I-5: Emulsion | |
|---|---|
| Recipe | Blend ratio |
| Stearic acid | 2.5 |
| Cetyl alcohol | 1.5 |
| Vaseline | 5.0 |
| Liquid paraffin | 10.0 |
| Polyoxyethylene (10 mol)monooleic ester | 2.0 |
| Polyethylene glycol 1500 | 3.0 |
| Triethanolamine | 1.0 |
| Carboxyvinyl polymer (tradename: Carbopol 941, B.F. Goodrich Chemical Co.) | 0.05 |
| Sorbitol | 0.01 |
| Sodium bisulfite | 0.01 |
| Ethyl paraben | 0.3 |
| Perfume | q.s. |
| Ion exchanged water | Bal. |

### Preparation Method

The carboxyvinyl polymer was dissolved in a small amount of the ion exchanged water (phase A). The polyethylene glycol 1500 and the triethanolamine were added to the remaining ion exchanged water, the sorbitol added, and the mixture heated to dissolve and held at 70°C (aqueous phase). The other components were mixed, heated to melt, and held at 70°C (oil phase). The oil phase was added to the aqueous phase and the mixture was preliminarily emulsified, then phase A was added and the mixture emulsified homogeneously by a homomixer. After emulsification, the emulsion was cooled to 30°C, while stirring well to obtain an emulsion.

| Example I-6: Emulsion | |
|---|---|
| Recipe | Blend ratio |
| Microcrystalline wax | 1.0 |
| Beeswax | 2.0 |
| Lanolin | 20.0 |
| Liquid paraffin | 10.0 |
| Squalane | 5.0 |
| Sorbitan sesquioleic ester | 4.0 |
| Polyoxyethene (20 mol) sorbitan monooleic ester | 1.0 |
| Propylene glycol | 7.0 |
| Mannitol | 5.0 |
| Sodium bisulfite | 0.01 |
| Ethyl paraben | 0.3 |
| Perfume | q.s. |
| Ion exchanged water | Bal. |

### Preparation Method

The mannitol and propylene glycol were added to the ion exchanged water and heated and held at 70°C (aqueous phase). The rest of the components were mixed and heated to melt and held at 70°C (oil phase). The oil phase was gradually added to the aqueous phase while stirring. The mixture was homogeneously emulsified by a homomixer, then was cooled to 30°C while stirring well.

| Example I-7: Jelly | |
|---|---|
| Recipe | Blend ratio |
| 95% Ethyl alcohol | 10.0 |
| Dipropylene glycol | 15.0 |
| Polyoxyethylene (50 mol) oleyl alcohol ether | 2.0 |
| Carboxyvinyl polymer (tradename: Carbopol 940, B.F. Goodrich Chemical) | 0.05 |
| Potassium hydroxide | 0.15 |
| Xylitol | 5.0 |
| Sodium bisulfite | 0.01 |
| Ethyl paraben | 0.3 |
| Perfume | q.s. |
| Ion exchanged water | Bal. |

### Preparation Method

The Carbopol 940 was homogeneously dissolved in the ion exchanged water and the polyoxyethylene (50 mol) oleyl alcohol ether was added to the aqueous phase. Next, the rest of the components were added, then the result was neutralized and thickened with sodium hydroxide and xylitol.

| Example I-8: Jelly | |
|---|---|
| Recipe | Blend ratio |
| (Phase A) | |
| 95% Ethyl alcohol | 10.0 |
| Polyoxyethylene (20 mol) octyldodecanol | 1.0 |
| Pantotjenyl ethyl ether | 0.1 |
| Methyl paraben | 0.15 |

| (Phase B) | |
|---|---|
| Potassium hydroxide | 0.1 |

| (Phase C) | |
|---|---|
| Glycerol | 5.0 |
| Dipropylene glycerol | 10.0 |
| Sodium bisulfite | 0.03 |
| Sorbitol | 0.05 |
| Carboxyvinyl polymer (tradename: Carbopol 940, B.F. Goodrich Chemical) | 0.2 |
| Ion exchanged water | Bal. |

### Preparation Method

The phase A and phase C were respectively uniformly dissolved and the phase A was added to the phase C to solubilize it. Next, the phase B was added, then the mixture filled to obtain a jelly.

| Example I-9: Pack | |
|---|---|
| Recipe | Blend ratio |
| (Phase A) | |
| Dipropylene glycol | 5.0 |
| Polyoxyethylene (60 mol) hydrogenated castor oil | 5.0 |

| (Phase B) | |
|---|---|
| Olive oil | 5.0 |
| Tocopheryl acetate | 0.2 |
| Ethyl paraben | 0.2 |
| Perfume | 0.2 |

| (Phase C) | |
|---|---|
| Xylitol | 1.0 |
| Sodium bisulfite | 0.03 |
| Polyvinyl alcohol (degree of saponification 90, degree of polymerization 2000) | 13.0 |
| Ethyl alcohol | 7.0 |
| Ion exchanged water | Bal. |

### Preparation Method

Phase A, phase B, and phase C were uniformly dissolved, then phase B was added to phase A to solubilize it. Next, phase C was added to this, then the mixture filled to obtain a pack.

| Example I-10: Solid Foundation | |
|---|---|
| Recipe | Blend ratio |
| Talc | 43.1 |
| Kaolin | 15.0 |
| Sericite | 10.0 |
| Zinc oxide | 7.0 |
| Titanium dioxide | 3.8 |
| Yellow iron oxide | 2.9 |
| Black iron oxide | 0.2 |
| Squalane | 8.0 |
| Isostearic acid | 4.0 |
| Polyoxyethylene sorbitan monooleic ester | 3.0 |
| Isocetyl octanate | 2.0 |
| Mannitol | 0.5 |
| Preservative | q.s. |
| Perfume | q.s. |

### Preparation Method

The powder components from the talc to the black iron oxide were sufficiently mixed by a blender, then the oil ingredients of the squalane to isocetyl octanate, the mannitol, preservative, and perfume were added and the resultant mixture was kneaded well and filled into a container for molding to obtain a solid foundation.

| Example I-11: Emulsion Type Foundation | |
|---|---|
| Recipe | Blend ratio |
| (Powder part) | |
| Titanium dioxide | 10.3 |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Aqueous phase) | |
|---|---|
| Ion exchanged water | 50.0 |
| 1,3-butylene glycol | 4.5 |
| Sorbitol | 1.5 |
| Sorbitan sesquioleic ester | 3.0 |
| Preservative | q.s. |
| Perfume | q.s. |

### Preparation Method

The aqueous phase was heated and stirred, then the sufficiently mixed and pulverized powder part was added and the mixture processed by a homomixer. Further, the heated and mixed oil phase was added and the mixture processed by a homomixer, then the perfume was added while stirring and the mixture cooled to room temperature to obtain an emulsion type foundation.

According to the first aspect of the present invention, it is possible to provide an external skin treatment composition for improving rough skin having a superior effect of improvement or prevention of various types of skin diseases accompanied with rough skin and rough skin, scaly skin, chapped skin etc. of healthy persons.

### Examples II-1 to II-3

The effect of promotion of recovery of the skin barrier function was evaluated by the following method. The results are shown in Fig. 1.

"Test of Effect of Promotion of Recovery of Skin Barrier Function"

The effect of a xylitol-containing sample in the process of recovery of the skin barrier function, destroyed by tape stripping of the skin, to the initial condition was evaluated by the following method using the TEWL as an indicator:

That is, using the inside part of the forearms of 10 male panelists, tape stripping was performed. After one hour, a xylitol-containing sample was applied, then the TEWL was measured along with time by a Tewameter TM-200 (Courage+Khazaka). A solution of 5% by weight of the evaluated substance using PEG300:Ethanol:Distilled water in a ratio of 1:3:1 as a substrate was used as the sample. Water was used as the control.

The recovery ratio was calculated from the values of the TEWL at different measurement times when the value of the TEWL after 1 hour after tape stripping was made 0% and the value of the TEWL after the tape stripping was made 100%. The effect of promotion of recovery of the TEWL of the samples was evaluated compared with the control. The results are shown in Fig. 1. In Fig. 1, the squares show the evaluated sample and the circles the control. The ordinate shows the TEWL recovery ratio (%), while the abscissa shows the time after the tape stripping (hr).

As will be understood from Fig. 1, the sample containing xylitol significantly promotes the recovery of the TEWL from a short time.

Examples of formulations of the present external skin treatment composition for promoting the skin barrier function will be aiven below.

| Example II-1: Cream | |
|---|---|
| Recipe | Blend ratio |
| Solid paraffin | 5.0 |
| Beeswax | 10.0 |
| Vaseline | 15.0 |
| Liquid paraffin | 41.0 |
| Glyceryl monostearic ester | 2.0 |
| Polyoxyethylene (20 mol) sorbitan monolauric ester | 2.2 |
| Soap powder | 0.1 |
| Sodium borate | 0.2 |
| Xylitol | 3.0 |
| Sodium bisulfite | 0.03 |
| Ethyl paraben | 0.3 |
| Perfume | q.s. |
| Ion exchanged water | Bal. |

### Preparation Method

The xylitol, soap powder, and sodium borate were added to the ion exchanged water, then heated and held at 70°C (aqueous phase). The rest of the components were mixed, heated to melt, and held at 70°C (oil phase). The oil phase was gradually added to the aqueous phase while stirring. The mixture was held at that temperature for a while after the total amount had finished being added to cause the reaction. Next, the resultant mixture was homogeneously emulsified by a homomixer, then cooled to 30°C, while stirring well to obtain a cream.

| Example II-2: Jelly | |
|---|---|
| Recipe | Blend ratio |
| 95% Ethanol | 10.0 |
| Dipropylene glycol | 15.0 |
| Polyoxyethylene (50 mol) oleyl alcohol | |
| ether | 3.0 |
| Carboxyvinyl polymer (tradename: Carbopol 940, B.F. Goodrich Chemical) | 0.05 |
| Sodium hydroxide | 0.15 |
| Xylitol | 5.0 |
| Sodium bisulfite | 0.01 |
| Ethyl paraben | 0.3 |
| Perfume | q.s. |
| Ion exchanged water | Bal. |

### Preparation Method

The carboxyvinyl polymer was uniformly dissolved in the ion exchanged water, while the polyoxyethylene (50 mol) oleyl alcohol ether was added to the aqueous phase. Next, the rest of the components were added, then the resultant mixture was neutralized and thickened with sodium hydroxide and xylitol to obtain a jelly.

| Example II-3: Pack | |
|---|---|
| Recipe | Blend ratio |
| (Phase A) | |
| Dipropylene glycol | 5.0 |
| Polyoxyethylene (60 mol) hydrogenated castor oil | 5.0 |

| (Phase B) | |
|---|---|
| Xylitol | 1.0 |
| Sodium bisulfite | 0.03 |
| Polyvinyl alcohol (degree of saponification 90, degree of polymerization 2000) | 13.0 |
| Ethanol | 7.0 |
| Ion exchanged water | Bal. |

### Preparation Method

Phase A, phase B, and phase C were uniformly dissolved, then phase B was added to phase A to solubilize it. Next, phase C was added to this, then the resultant mixture was filled to obtain a pack.

According to the second aspect of the present invention, it is possible to provide a superior promoter for recovery of skin barrier function capable of promoting the recovery of the barrier function of the skin.

## Claims

1. An external skin treatment composition comprising, as an effective component, a sugar alcohol having 5 or 6 carbon atoms and a carrier therefor.

2. An external skin treatment composition as claimed in claim 1, wherein a formulation amount of the sugar alchol, based upon the total amount of the composition, is 0.005 to 30% by weight.

3. An external skin treatment composition as claimed in claim 1, wherein said sugar is at least one compound selected from the group consisting of xylitol, sorbitol and mannitol.

4. An external skin treatment composition as claimed in claim 1 for improving and preventing roughening of skin.

5. An external skin treatment composition as claimed in claim 4, wherein the formulation amount of the sugar alcohol is 0.01 to 10% by weight, based upon the total amount of the composition.

6. An external skin treatment composition as claimed in claim 4, wherein the sugar alcohol is at least one compound selected from the group consisting of xylitol, sorbitol and mannitol.

7. An external skin treatment composition as claimed in claim 1 for promoting recovery of skin barrier function.

8. An external skin treatment composition as claimed in claim 7, wherein the formulation amount of the sugar alcohol is 0.1 to 20% by weight, based upon the total amount of the composition.

9. An external skin treatment composition as claimed in claim 7, wherein the sugar alcohol is at least one compound selected from the group consisting of xylitol, sorbitol and mannitol.
